# EUROPEAN PATENT APPLICATION

(11) **EP 1 728 438 A1**
(43) Date of publication of application: **06.12.2006**
(21) Application number: 05011785.2
(22) Date of filing: 01.06.2005
(51) Int. Cl.: A23K 1/00, A23K 1/16

(54) **Feedstuff**

(71) Applicant: NOLabs AB, 252 21 Helsingborg (SE)
(72) Inventor: Peters, Tor, 8703 Erlenbach (CH)
(74) Representative: Asketorp, Göran

(57) **Abstract**

A feedstuff additive product and a feedstuff configured for growth promoting, anti-pathogenic, antimicrobial and/or anti-viral effect, and a manufacturing process thereof,comprising a nitric oxide (NO) eluting polymer configured for eluting a non-toxic amount of nitrogen oxide (NO) to an animal, such as a mammal, at contact with moisture.

## Description

### Field of the Invention

This invention pertains in general to the field of feedstuff. More particularly the invention relates to a feedstuff, and a process for manufacturing of said feedstuff, said feedstuff being configured for growth promoting, anti-pathogenic, anti-microbial and anti-viral effect.

### Background of the Invention

Animals, such as pigs, poultry, cattle, horses, veal calves, ostriches, fish, etc., are grown intensively for the production of meat, fish and eggs. These animals are fed diets containing a variety of materials of animal and/or vegetable origin to supply energy and protein, as well as to fulfil other requirements. Most of the feedstuff that is consumed is produced commercially, but a significant part is produced on the farm and fed directly. The feedstuff is often supplemented with vitamins and minerals to meet the animal's nutrient requirements. The use of industrially produced enzymes in these feedstuffs has now almost become common practice. The enzymes are used to promote growth and feedstuff conversion, and to reduce the environmental pollution produced by manure from pigs, poultry and fish. However, costs related to feedstuff are the most important cost factor in animal production.

Also, other animals and pets, such as dogs, cats, horses, etc., are in need of feedstuff that may treat pathogenic, microbial, and viral attacks, and induce promotion of growth.

Furthermore, antibiotics have been used in feedstuff during a long time to also achieve an anti-microbial effect. While antibiotics have been routinely added to animal feedstuff, the resistance of bacteria, which are pathogenic to humans, against antibiotics has been increasing rapidly. This has made it more difficult to cure people from bacterial infections, and the widespread use of antibiotics in animal feedstuff has been blamed by various experts for the acceleration of resistance to various antibiotics. This has led to a ban on the use of most antibiotics as ingredients in animal feedstuff in the European Union. It is likely that other countries will follow this example due to pressure from consumer and healthcare organizations.

It is known that nitric oxide (NO) provides an alternative to conventional therapies, such as antibiotics. Nitric oxide is a highly reactive molecule that is involved in many cell functions. In fact, nitric oxide plays a crucial role in the immune system and is utilized as an effector molecule by macrophages to protect itself against a number of pathogens, such as fungi, viruses, bacteria etc., and general microbial invasion. This improvement of healing is partly caused by NO inhibiting the activation or aggregation of blood platelets, and also by NO causing a reduction of inflammatory processes, for example at the site of an implant.

NO is also known to have an anti-pathogenic, especially an anti-viral, effect, and furthermore NO has an anti-cancerous effect, as it is cytotoxic and cytostatic in therapeutic concentrations, i.e. it has among other effects tumoricidal and bacteriocidal effects. NO has for instance cytotoxic effects on human haematological malignant cells from patients with leukaemia or lymphoma, whereby NO may be used as a chemotherapeutic agent for treating such haematological disorders, even when the cells have become resistant to conventional anti-cancer drugs. This anti-pathogenic and anti-tumour effect of NO is taken advantage of by the present invention, without causing adverse effects as for instance many other anti-cancer drugs.

However, due to the short half-life of NO, in the range of a few seconds, it has hitherto been difficult to treat viral, bacteria, virus, fungi or yeast infections with NO. This is also because NO is actually toxic in high concentrations and has negative effects when applied in too large amounts to the body. NO is also a vasodilator, and too large amounts of NO introduced into the body may cause a complete collapse of the circulatory system. On the other hand, NO has a very short half-life of fractions of a second up to a few seconds, once it is released. Hence, administration limitations due to short half-life and toxicity of NO have been limiting factors in the use of NO in the field of anti-pathogenic and anti-cancerous treatment.

In recent years research has been directed to polymers with the capability of releasing nitrogen oxide at contact with water. Such polymers are for example polyalkyleneimines, such as L-PEI (Linear PolyEthyleneImine) and B-PEI (Branched PolyEthyleneImine), which polymers have the advantage of being biocompatible, after the release of nitrogen oxide.

Other examples of NO eluting polymers are given in US-5,770,645, wherein polymers derivatized with at least one -NOₓ group per 1200 atomic mass unit of the polymer are disclosed, X being one or two. One example is an S-nitrosylated polymer, which is prepared by reacting a polythiolated polymer with a nitrosylating agent under conditions suitable for nitrosylating free thiol groups.

Akron University has developed NO-eluting L-PEI molecules that can be nano-spun onto the surface of medical devices to be permanently implanted in the body, such as implanted grafts, showing significant improvement of the healing process and reduced inflammation when implanting such devices. According to US-6,737,447, a coating arranged on medical devices provides nitric oxide delivery at contact with water and using nanofibers of linear poly(ethylenimine)-diazeniumdiolate. Linear poly(ethylenimine)diazeniumdiolate releases nitric oxide (NO) in a controlled manner to tissues and organs to aid the healing process and to prevent injury to tissues at risk of injury. Electrospun nano-fibers of linear poly(ethylenimine) diazeniumdiolate deliver therapeutic levels of NO to the tissues surrounding a medical device while minimizing the alteration of the properties of the device. A nanofiber coating, because of the small size and large surface area per unit mass of the nanofibers, provides a large surface area per unit mass while minimizing changes in other properties of the device.

In the prior art, feedstuffs have been disclosed that obtain NO from propionic bacteria, such as in disclosed in US20050036996. These kind of feedstuffs are associated with complicated and intricate manufacturing processes, including culturing, harvesting, etc., and the present invention is more cost effective than said processes.

The industry of feedstuff is therefore interested in alternative additives with growth promoting, anti-pathogenic, anti-microbial and anti-viral effects, without any therapeutic side effects for consumers, which additives may be manufactured in a cost effective way.

However, all the above-mentioned publications are silent concerning an improvement of the present technology in respect of using an NO releasing polymer in feedstuff to obtain growth promoting, anti-pathogenic, anti-microbial and anti-viral effects.

Hence, an improved additive for feedstuff would be advantageous, which presents growth promoting, anti-pathogenic, anti-microbial and anti-viral effects, without any therapeutic side effects for consumers, and which does not induce resistance against the active pharmaceutical substance, and which provides improved circulation in form of a vasodilating effect, and has fast inset of effect.

### Summary of the Invention

Accordingly, the present invention preferably seeks to mitigate, alleviate or eliminate one or more of the above-identified deficiencies in the art and disadvantages singly or in any combination and solves at least the problems mentioned above, by providing a device according to the appended patent claims.

According to one aspect of the invention, a feedstuff additive is provided that allows for growth promoting, anti-pathogenic, anti-microbial and anti-viral effects, without any therapeutic side effects in consumers. The feedstuff comprises a nitric oxide (NO) eluting polymer, such that a therapeutic dose of nitric oxide is eluted from said nitric oxide eluting polymer to an area.

According to another aspect of the invention, a manufacturing process for such a feedstuff is provided, wherein the process is a process for forming a feedstuff that allows for elution of NO in the gastric area of a mammal. The process comprises selecting a plurality of nitric oxide eluting polymeric particles, such as nano fibres, fibres, nano particles, or microspeheres, and deploying said nitric oxide eluting particles into forms such as nano-particles, micro-spheres, or powder to be comprised in said feedstuff.

According to still another aspect of the present invention a feedstuff comprising said feedstuff additive is provided that allows for growth promoting, anti-pathogenic, anti-microbial and anti-viral effects, without any therapeutic side effects in consumers.

The present invention has at least the advantage over the prior art that it presents the possibility to provide growth promoting, anti-pathogenic, anti-microbial and anti-viral effects, without any therapeutic side effects in consumers, does not develop resistance against the active pharmaceutical substance, provides improved circulation in form of a vasodilating effect, and has fast inset of effect, by the exposure of an area to NO, by elution of NO from an NO eluting polymer.

### Brief Description of the Drawings

These and other aspects, features and advantages of which the invention is capable of will be apparent and elucidated from the following description of embodiments of the present invention, reference being made to the accompanying drawings, in which
Fig. 1 is a schematic illustration of a nano-particles, or micro-spheres, according to the invention,
Fig. 2 is a schematic illustration of a drink, and according to the invention.

### Description of Embodiments

The following description focuses on embodiments of the present invention applicable to a feedstuff additive, and a feedstuff, comprising nano-particles, or micro-spheres, which allow for exposure of the gastric area to NO, obtained through elution of NO from an NO eluting polymer. The feedstuff according to the present invention may be fed to all kinds of animals, such as pigs, poultry, cattle, horses, veal calves, fish, dogs, cats, ostriches, that are in need of growth promoting, anti-pathogenic, anti-microbial and anti-viral effect.

With regard to nitric oxide (nitrogen monoxide, NO), its physiological and pharmacological roles have attracted much attention and thus have been studied. NO is in the living body synthesized from arginine as the substrate by nitric oxide synthase (NOS). NOS is classified into a constitutive enzyme, cNOS, which is present even in the normal state of a living body and an inducible enzyme, iNOS, which is produced in a large amount in response to a certain stimulus. It is known that, as compared with the concentration of NO produced by cNOS, the concentration of NO produced by iNOS is 2 to 3 orders higher (i.e. up to 1000 times higher), and that iNOS produces an extremely large amount of NO.

In the case of the generation of a large amount of NO as in the case of the production by iNOS, it is known that NO reacts with active oxygen to attack i.a. exogenous microorganisms and cancer cells, but may also cause inflammation and tissue injury. On the other hand, in the case of the generation of a small amount of NO as in the case of the production by cNOS, it is considered that NO takes charge of various protective actions for a living body through cyclic GMP (cGMP), such as vasodilatory action, improvement of the blood circulation, antiplatelet-aggregating action, antibacterial action, anticancer action, acceleration of the absorption at the digestive tract, renal function regulation, neurotransmitting action, erection (reproduction), learning, appetite, and the like. Heretofore, inhibitors of the enzymatic activity of NOS have been examined for the purpose of preventing inflammation and tissue injury, which are considered to be attributable to NO generated in a large amount in a living body. However, the promotion of the enzymatic activity (or expressed amount) of NOS (in particular, cNOS) has not been examined for the purpose of exhibiting various protective actions for a living body by promoting the enzymatic activity of NOS and producing NO appropriately.

In recent years, research has been directed to polymers with the capability of releasing nitrogen oxide when getting in contact with water. Such polymers are for example polyalkyleneimines, such as L-PEI (Linear PolyEthyleneImine) and B-PEI (Branched PolyEthyleneImine), which polymers have the advantage of being biocompatible, after the release of nitrogen oxide.

Such polymers may be manufactured by electro spinning. Electro spinning is a process by which a suspended polymer is charged. At a characteristic voltage, a fine jet of polymer releases from the surface in response to the tensile forces generated by interaction by an applied electric field with the electrical charge carried by the jet. This process produces a bundle of polymer fibres, such as nano-fibres. This jet of polymer fibres may be directed to a surface to be treated.

Furthermore, US 6,382,526, US 6,520,425, and US 6,695,992 disclose processes and apparatuses for the production of such polymeric fibres. These techniques are generally based on gas stream spinning, also known within the fiber forming industry as air spinning, of liquids and/or solutions capable of forming fibers.

Other examples of NO eluting polymers are given in US-5,770,645, wherein polymers derivatized with at least one -NO_{X} group per 1200 atomic mass unit of the polymer are disclosed, X being one or two. One example is an S-nitrosylated polymer, which is prepared by reacting a polythiolated polymer with a nitrosylating agent under conditions suitable for nitrosylating free thiol groups.

Akron University has developed NO-eluting L-PEI molecules that can be nano-spun onto the surface of medical devices such as grafts to be implanted, showing significant improvement of the healing process and reduced inflammation when implanting such devices. According to US-6,737,447, a coating for permanently implanted medical devices provides nitric oxide delivery using nanofibers of linear poly(ethylenimine)-diazeniumdiolate. Linear poly(ethylenimine)diazeniumdiolate releases nitric oxide (NO) in a controlled manner. Another advantage of L-PEI is that NO is released without release of any secondary products that could lead to undesired side effects.

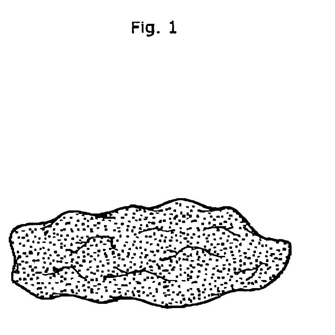

In an embodiment of the invention, according to Fig. 1, the feedstuff additive is embodied in form of nano-particles, or micro spheres. This feedstuff additive may be integrated in normal feedstuff. The term "normal feedstuff" is in the present invention intended to be interpreted as all kinds of feedstuff that are used in the area of feeding mammals. These nano-particles, or micro-spheres, may be formed from the NO-eluting polymers. The nano-particles or micro-spheres are, after being mixed with normal feedstuff, swallowed for accessing the gastro-intestinal tract of a body. When the nano-particles, or micro-spheres, get in contact with the moisture in the stomach, the NO-eluting polymer starts to elute NO on said gastro-intestinal tract, such as the stomach. At this point an effective elution of NO is initiated, which elution brings about a growth promoting, anti-pathogenic, anti-microbial and/or anti-viral effect.

The nano-particles, or micro-spheres, may alternatively be fed directly, without first mixing them with the normal feedstuff.

In another embodiment of the invention, the nano-particles, or micro-spheres, formed from the NO-eluting polymers, are encapsulated, or integrated, in any suitable material, such as polyvinylacetates, polylacticacids, starch, cellulose, polyhydroxyalkanoates, polycaprolactone, polyvinylalcohol, protein based plastics, gelatine, biogradable or biocompatible polymers, and other soluble plastics. The integration of, or encapsulation in, these materials is performed to regulate and/or control the elution of NO in the stomach of the mammal, and to provide continuous exposure of the gastrointestinal tract to NO. The encapsulation may be such that the material breaks by the movement of the stomach, or dissolves in the aqueous acidic environment in the stomach.

In yet another embodiment of the present invention, the feedstuff additive, and a feedstuff comprising the same, comprises the NO eluting polymer in form of a powder, said powder being obtained by grounding or pulverizing the NO-eluting polymer according to the invention.

When the nano-particles, or micro-spheres, or powder, according to this embodiment, gets in contact with the moisture in the stomach, they/it start/starts to elute NO in the gastro-intestinal tract. At this point an effective elution of NO is initiated, which results in growth promoting, anti-pathogenic, anti-microbial and anti-viral effects, without any therapeutic side effects for the animals getting fed by said feedstuff, or the consumers eating said animals.

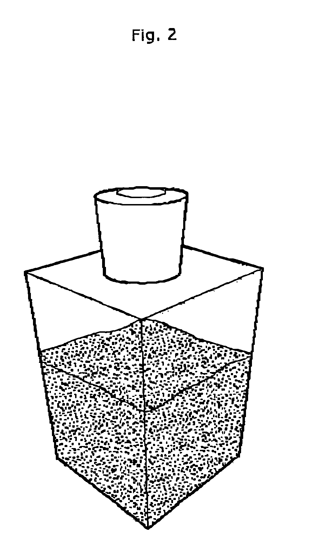

In still another embodiment the nano-particles, or micro-spheres, are encapsulated in a material, which does not dissolve in water but is dissolved in an acid environment, to form a product, which may be integrated in a suitable liquid, said liquid being drinkable, according to Fig. 2, When the liquid reaches the stomach the encapsulation material is dissolved by the hydrogen chloride acid in the stomach and the nano-particles, or micro-spheres start to elute NO. At this point an effective NO elution is initiated, which results in growth promoting, anti-pathogenic, anti-microbial and/or anti-viral effects, without any therapeutic side effects in consumers.

In another embodiment of the present invention the nano-particles, or micro-spheres, or ground/pulverized NO-eluting polymer, may be encapsulated in a material, such as gelatine, starch, cellulose etc, to provide a capsule to be swallowed. When the thus obtained capsule reaches the stomach, the gelatine dissolves and the nano-particles, or micro-spheres, start to elute NO.

In still another embodiment of the present invention the nano-particles, or micro-spheres, or ground/pulverized NO-eluting polymer may be encapsulated in a material that is insoluble in the acidic environment of the stomach. In this embodiment the capsule is instead dissolved in the alcaline environment in the lower part of the gastro-intestinal tract.

In another embodiment of the present invention the nano-particles, or micro-spheres, or ground/pulverized NO-eluting polymer are/is compressed into a pill, tablet or pellet, which pill, tablet or pellet may be swallowed by the mammal. When the pill, tablet, or pellet, reaches the stomach, an effective elution of NO is initiated, which results in growth promoting, anti-pathogenic, anti-microbial and/or anti-viral effects, without any therapeutic side effects in consumers.

In yet another embodiment of the present invention the NO-eluting feedstuff may be combined with, or acting as a booster for other pharmaceuticals, minerals, hormones, and vitamins. This embodiment presents a feedstuff with the advantage of combining several therapeutic effects, of significant value, in one treatment. Hence, a synergetic effect, as explained blow, may be achieved by such feedstuff when NO is eluted from the feedstuff in combination with other pharmaceuticals, minerals, hormones, and vitamins. NO has for instance a vasodilatory effect on the gastro-intestinal tract where the feedstuff elutes NO, which may facilitate the uptake of the combined substance, such as pharmaceutical, mineral, hormone, and/or vitamin. Vasodilated tissue may be more susceptible to certain medications and thus more easily treated by the medical preparations and still NO has in addition to that the anti-inflamatory, anti-bacterial etc. effect. Hence, an unexpected surprisingly effective treatment is provided by feeding the mammal with this kind of feedstuff, comprising said combination.

In another embodiment of the feedstuff additive, and feedstuff, according to the present invention, the nano-particles, or micro-spheres, are integrated in a gel. It may also be integrated in a hydrogel, which is mixed directly before it is fed to the mammal. This gel is then swallowed, and the NO eluting polymer elutes NO when the gel, or hydrogel, reaches the gastro-intestinal tract. This embodiment has the advantage of being able to penetrate pockets and corners in the gastrointestinal tract for closer elution of NO.

In another embodiment of the present invention the nano-particles, or micro-spheres, of the polymers in the present invention, may be encapsulated in a material that breaks when being exposed to stresses from chewing. The materials used to encapsulate these nano-particles, or micro-spheres, may be chosen from the group comprising polyvinylacetates, polylacticacids, starch, cellulose, polyhydroxyalkanoates, polyesters, polycaprolactone, polyvinylalcohol, protein based plastics, gelatine, biogradable polymers, and other soluble plastics. When the nano-particles, or micro-spheres, are chewed, the nano-particles, or micro-spheres, start to elute NO. The encapsulation materials provide the possibility to regulate and control the elution of NO. This embodiment has the advantages that it provides another option of another way to obtain a continuous dosage of NO, is easy to apply, the treatment effect covers the whole oral cavity, and it is easy to manufacture.

In another embodiment the nano-particles, or micro-spheres, of the polymers in the present invention, may be integrated in a film. When the film is put in the mouth of the mammal the nano-particles, or micro-spheres, of NO-eluting polymer elutes NO that is swallowed, and said swallowed NO results in growth promoting, anti-pathogenic, anti-microbial and anti-viral effects, without any therapeutic side effects in consumers. The encapsulation materials, according to above, provides the possibility to regulate and control the elution of NO. The film may also be swallowed before dissolving, thus effecting directly the gastro-intestinal tract.

In yet another embodiment of the present invention the feedstuff comprises NO eluting polymer in form of a foam or cream.

When the NO-eluting feedstuff, comprising the NO-eluting feedstuff additive according to the present invention, gets in contact with the moisture in the stomach, the NO-eluting feedstuff starts to release NO to the gastro-intestinal tract. This feedstuff does not develop resistance against nitric oxide (NO), is easy to apply, has fast inset of effect, and provides growth promoting, anti-pathogenic, anti-microbial and/or anti-viral effects, without any therapeutic side effects in consumers.

In another embodiment of the feedstuff according to the present invention comprises a basic carrier material, which basic material is integrated, or covered, with the nano-fibres, nano-particles, powder and/or micro-spheres of NO-eluting polymer according to the invention. This basic carrier material is then mixed with normal feedstuff to obtain the feedstuff according to the present invention. When the basic material, for example in form of small granules, reaches the gastro-intestinal tract, the nano-fibres, nano-particles, powder and/or micro-spheres of NO-eluting polymer start to elute NO on the area to be treated. At this point an effective elution of NO is initiated, which results in growth promoting, anti-pathogenic, anti-microbial and/or anti-viral effects, without any therapeutic side effects in consumers. The granules, according to this embodiment of the invention, then passes through the gastrointestinal tract, and exit the body in the faeces.

The basic material, integrated in the feedstuff additive or feedstuff according to the invention, may be polyesters, polyamides, polyethers, polyurethanes, polycarbonates, polyvinylacetates, polylacticacids, starch, cellulose, polyhydroxyalkanoates, polypropylene, cotton, polyesters, polycaprolactone, polyvinylalcohol, polyacrylonitrile, polystyrene, poly(acrylic acid), polypropylene, protein based plastics, gelatine, and other biocompatible polymers. The NO-eluting polymer, such as L-PEI, may be integrated in, spun together with, or spun on top of, any of these materials.

The feedstuff additive or feedstuff according to the present invention elutes nitric oxide (NO) from said eluting polymer in a therapeutic dose, such as between 0.001 to 100 ppm, such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 91, 92, 93, 94, 95, 96, 97, 98, 99, or 100 ppm.

The NO-eluting polymers in the feedstuff additives or feedstuffs according to the present invention may be combined with silver, such as hydroactivated silver. The integration of silver in the feedstuffs according to the present invention gives the treatment an extra boost. The silver may be releasable from the feedstuffs in the form of silver ions.

In the embodiments of the present invention it may be suitable to control or regulate the time span of NO release from the feedstuff additive or feedstuff according to the invention. This may be accomplished by integrating other polymers or materials in said feedstuff additive or feedstuff. These polymers or materials may be chosen from any suitable material or polymer, such as polyvinylacetates, polylacticacids, starch, cellulose, polyhydroxyalkanoates, polyesters, polycaprolactone, polyvinylalcohol, protein based plastics, gelatine, biogradable polymers, and other soluble plastics.

The feedstuff additive according to the present invention may be manufactured by, for example electro spinning of L-PEI or other polymers comprising L-PEI or being arranged in combination with L-PEI. L-PEI is then charged at a characteristic voltage, and a fine jet of L-PEI releases as a bundle of L-PEI polymer fibres. This jet of polymer fibres may be directed to a surface to be treated. The surface to be treated may for example be any suitable material in respect of a feedstuff according to the present invention. The electro spun fibres of L-PEI then attach on said material and form a coating/layer of L-PEI on the materials comprised in the feedstuff according to the invention. Such fibres are also easily further processed to other forms, such as the above mentioned powder, which simply is obtainable by applying a shredder or blender type apparatus to the fibres until a powder of desired granulation size is received.

The NO-eluting polymers according to the present invention may also be spun directly on a normal feedstuff.

It is possible to electro spin other NO-eluting polymers, according to above, on the materials comprised in the feedstuff according to the invention while still being within the scope of the present invention.

In an embodiment, the NO-eluting polymers are electro spun in such way that pure NO-eluting polymer fibres may be obtained.

Gas stream spinning, or air spinning, of said NO-eluting polymers onto the material comprised in the feedstuff according to the present invention is also within the scope of an embodiment of the manufacturing method according to the present invention.

The manufacturing process according to the present invention presents the advantages of providing feedstuffs with large contact surface of the NO-eluting polymer fibres with moisture in the gastro-intestinal tract, effective use of NO-eluting polymer, and a cost effective way of producing the feedstuff according to the present invention.

The elements and components of the embodiments according to the invention may be physically, functionally, and logically implemented in any suitable way. Indeed, the functionality may be implemented in a single unit, in a plurality of units, or as part of other functional units.

Although the present invention has been described above with reference to specific embodiments, it is not intended to be limited to the specific form set forth herein. Rather, the invention is limited only by the accompanying claims and other embodiments than those specifically described above are equally possible within the scope of these appended claims.

In the description and the claims of the present invention, the term "comprises/comprising" does not exclude the presence of other elements or steps. Furthermore, although individually listed, a plurality of means, elements or method steps may be implemented. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality. Reference signs in the claims are provided merely as a clarifying example and shall not be construed as limiting the scope of the claims in any way.

## Claims

1. A feedstuff additive product configured for growth promoting, anti-pathogenic, anti-microbial and/or anti-viral effect, when added to feedstuff intended for animals, such as mammals, c h a r a c t e r i z e d in that
said product comprises a nitric oxide (NO) eluting polymer configured for eluting a non-toxic amount of nitrogen oxide (NO), said amount providing said promotion of growth, anti-pathogenic, anti-microbial, and/or anti-viral effect when added to said feedstuff and consumed or ingested by said animal.

2. The feedstuff additive product according to claim 1, wherein said polymer is selected from the group comprising polyalkyleneimines, S-nitrosylated polymer, and poly(alkylenimine)diazeniumdiolates, or any combinations thereof, loaded with said NO for said eluting.

3. The feedstuff additive product according to claim 1, wherein said polymer is L-PEI (linear polyethyleneimine), loaded with nitric oxide(NO), arranged for release of the nitric oxide (NO) at a gastro-intestinal tract of a mammal.

4. The feedstuff additive product according to claim 1, in a form selected from the group consisting of powder, nano-particles or micro-spheres, pill, tablet, pellet, drink, gel, hydrogel, foam, cream, granules, film, and capsule, or combinations thereof.

5. The feedstuff additive product according to claim 4, wherein said product is encapsulated, or integrated, in a material, selected from the group consisting of polyvinylacetates, polylacticacids, starch, cellulose, polyhydroxyalkanoates, polyesters, polycaprolactone, polyvinylalcohol, protein based plastics, and/or gelatine, and combinations thereof.

6. The feedstuff additive product according to claim 4 or 5, wherein said product is integrated in a drink, gel, hydrogel, film, or foam, or combinations thereof.

7. The feedstuff additive product according to claim 4, 5, or 6, wherein said granules are of a material, selected from the group consisting of polyesters, polyamides, polyethers, polyurethanes, polycarbonates, polyvinylacetates, polylacticacids, starch, cellulose, polyhydroxyalkanoates, polypropylene, cotton, polyesters, polycaprolactone, polyvinylalcohol, polyacrylonitrile, polystyrene, poly(acrylic acid), polypropylene, protein based plastics, gelatine, and other biocompatible polymers, and combinations thereof, integrated, and/or covered, with said NO-eluting polymer.

8. The feedstuff additive product according to claim 1, wherein said product is partly disintegrable when subjected to moisture or water.

9. The feedstuff additive product according to claim 1, wherein said product comprises silver.

10. The feedstuff additive product according to claim 1, wherein said polymer, in use during said consumption, is acting as a booster for pharmaceuticals, vitamins, minerals, hormones, and/or drugs, or combinations thereof when eluting said amount of NO.

11. A method of manufacturing a product according to any of claims 1 to 10, comprising:
spinning a polymer, having nitric oxide eluting property when contacted with water, by electro spinning or gas stream spinning, to form polymeric particles, such as nano fibres, nano particles of micro speres,
said polymeric particles comprise a nitric oxide (NO) eluting polymer configured for eluting a non-toxic amount of nitrogen oxide (NO), said amount providing promotion of growth, anti-pathogenic, anti-microbial, and/or anti-viral effect when added to a feedstuff and ingested by an animal.

12. Use of a nitric oxide (NO) eluting polymer for the manufacture of a feedstuff additive product for obtaining growth promoting, anti-pathogenic, anti-microbial, and/or anti-viral effect, wherein
nitric oxide, during said manufacture, is loaded to said feedstuff additive product so that said feedstuff additive product is configured to elute nitric oxide (NO) from said eluting polymer in a non-toxic dose when consumed by an animal.

13. Use of a feedstuff additive product according to claim 1 as an integrated part in a feedstuff.

14. Use according to claims 12 and 13, wherein said therapeutic dose is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 91, 92, 93, 94, 95, 96, 97, 98, 99, or 100 ppm.

15. A feedstuff configured for growth promoting, anti-pathogenic, anti-microbial and/or anti-viral effect, comprising the feedstuff additive according to claim 1.

16. A method of obtaining growth promoting, anti-pathogenic, anti-microbial and/or anti-viral effect for animals, such as mammals, by means of a feedstuff additive product comprising a nitric oxide (NO) eluting polymer configured for eluting a non-toxic amount of nitrogen oxide (NO) at contact with moisture, comprising exposing a gastro-intestinal tract of said animal to said amount of nitric oxide from said feedstuff additive product when said polymer in use elutes nitrogen oxide (NO) to said gastro-intestinal tract.

17. The method according to claim 16, wherein said method comprises applying said feedstuff additive product in the form of nano-particles or micro-spheres, a pill, a tablet, a pellet, a drink, a gel, a hydrogel, a foam, a cream, granules, and/or a capsule to said gastro-intestinal tract.

18. The method according to claim 17, wherein application of said feedstuff additive product is performed as an together, as a mixture or separately, with a normal feedstuff.
